# EUROPEAN PATENT APPLICATION

(11) **EP 4 190 395 A1**
(43) Date of publication of application: **07.06.2023**
(21) Application number: 21861588.8
(22) Date of filing: 25.08.2021
(51) Int. Cl.: A61N 1/30, A61F 9/007

(54) **MEDICINE ADMINISTERING TOOL**

(30) Priority: 28.08.2020 JP 2020144755
(71) Applicant: TERUMO Kabushiki Kaisha, Tokyo 151-0072 (JP)
(72) Inventor: SHIMAMURA, Yoshihisa, Ashigarakami-gun, Kanagawa 259-0151 (JP); HYAKKAN, Yusuke, Ashigarakami-gun, Kanagawa 259-0151 (JP); TANAKA, Ryo, Ashigarakami-gun, Kanagawa 259-0151 (JP); ITO, Eriko, Ashigarakami-gun, Kanagawa 259-0151 (JP); IWASE, Yoichiro, Ashigarakami-gun, Kanagawa 259-0151 (JP)
(74) Representative: Casalonga
(86) International application number: PCT/JP2021/031076
(87) International publication number: WO 2022/045164

(57) **Abstract**

A medicine administration tool (24a) constituting a transocular iontophoresis device (10a) includes a tubular member (40a), an inner member (42a), and a support member (66) that movably supports the inner member with respect to the tubular member. The tubular member and the inner member respectively have a first lower end (47) and a second lower end (59) facing an eyeball (14). Further, the inner member is accommodated in a hollow of the tubular member. As a result, a medicine accommodating chamber (52) for accommodating medicine (MD) is formed between the inner member and the tubular member.

## Description

### Technical Field

The present invention relates to a medicine administration tool constituting a transocular iontophoresis device, and more particularly to a medicine administration tool for allowing medicine to permeate from an eyeball using iontophoresis.

### Background Art

As an external treatment method for eye diseases, administration of eye drops and intravitreal injection are known. However, in a case of eye drops, an amount of effective medicine that penetrates from the cornea and migrates into the anterior chamber is about 1/10000 of the dose. In addition, an injection needle is inserted into an eyeball in intravitreal injection, and thus, psychological burden on a patient tends to increase.

Thus, as described in JP 2011-505917 T, it has been attempted to administer medicine for treating eye diseases using iontophoresis. In this case, it is expected that an electric circuit is formed between a first electrode attached to the skin of a human body and a second electrode provided in a medicine administration tool covering the eyeball, and a sufficient amount of the medicine can penetrate the eyeball by moving the charged medicine according to the principle of electrophoresis.

### Summary of Invention

The device described in JP 2011-505917 T includes a reservoir, a foam holder provided inside the reservoir and holding medicine, and a soft plastic material provided at an end of the reservoir facing an eyeball and in contact with the eyeball. The medicine is supplied into a range covered with the soft plastic material.

Here, a degree of curvature of the eyeball varies among individuals. For this reason, a clearance is formed between an edge portion of the soft plastic material and the eyeball depending on the patient, and there is a concern that the medicine leaks from the clearance. If such a situation occurs, it is difficult to allow a sufficient amount of the medicine to permeate the eye.

A general object of the present invention is to provide a medicine administration tool for a transocular iontophoresis device.

A main object of the present invention is to provide a medicine administration tool capable of avoiding leakage of medicine regardless of a degree of curvature of an eyeball.

According to an embodiment of the present invention, there is provided a medicine administration tool to be used in a transocular iontophoresis device that forms an electric circuit between a first electrode and a second electrode and supplies medicine to an eyeball, the medicine administration tool including:
a tubular member having a first lower end facing the eyeball;
an inner member having a second lower end facing the eyeball, the inner member being accommodated inside a hollow of the tubular member and forming a medicine accommodating chamber for accommodating the medicine between the inner member and the tubular member; and
a support member movably supporting the inner member with respect to the tubular member.

According to the present invention, the inner member moves relative to the tubular member according to a degree of curvature of the eyeball. Thus, the sclera is covered with the tubular member and the cornea is covered with the inner member without being affected by the degree of curvature (individual difference in shape) of the eyeball. Thus, the medicine is prevented from leaking from a clearance, which is formed between the tubular member and the sclera, to outside of the medicine administration tool. It is therefore possible to effectively administer the medicine.

In addition, the medicine is prevented from reaching the cornea from a clearance that is formed between the inner member and the cornea. This avoids the cornea from becoming a conductive path, thus protecting the cornea.

### Brief Description of Drawings

Fig. 1 is a schematic configuration diagram of a transocular iontophoresis device including a medicine administration tool according to a first embodiment of the present invention.
Fig. 2 is an enlarged front view of a passive electrode illustrated in Fig. 1.
Fig. 3 is a schematic exploded perspective view of the medicine administration tool illustrated in Fig. 1.
Fig. 4 is a schematic longitudinal cross-sectional view of the medicine administration tool illustrated in Fig. 1.
Fig. 5 is a schematic overall perspective view of a bush illustrated in Figs. 3 and 4.
Fig. 6 is an enlarged view of a main part in a longitudinal section of the medicine administration tool.
Fig. 7A is an enlarged cross-sectional view of a main part when the medicine administration tool is placed on an eyeball having a large degree of curvature, and Fig. 7B is an enlarged cross-sectional view of a main part when the medicine administration tool is placed on an eyeball having a small degree of curvature.
Fig. 8 is a schematic longitudinal cross-sectional view of a medicine administration tool according to a second embodiment of the present invention.
Fig. 9 is a schematic longitudinal cross-sectional view of a medicine administration tool according to a third embodiment of the present invention.
Fig. 10 is a schematic longitudinal cross-sectional view of a medicine administration tool according to a fourth embodiment of the present invention.
Fig. 11 is a schematic longitudinal cross-sectional view of a medicine administration tool according to a fifth embodiment of the present invention.
Fig. 12 is an enlarged cross-sectional view of a main part of the medicine administration tool in which a second protective material is a film-shaped body.
Fig. 13 is an enlarged cross-sectional view of a main part of the medicine administration tool in which the second protective material is a bead cushion.
Fig. 14 is an enlarged cross-sectional view of a main part of the medicine administration tool in which the second protective material is a soft polymer.

### Description of Embodiments

Hereinafter, preferred embodiments of a medicine administration tool according to the present invention in relation to a transocular iontophoresis device will be described in detail with reference to the accompanying drawings.

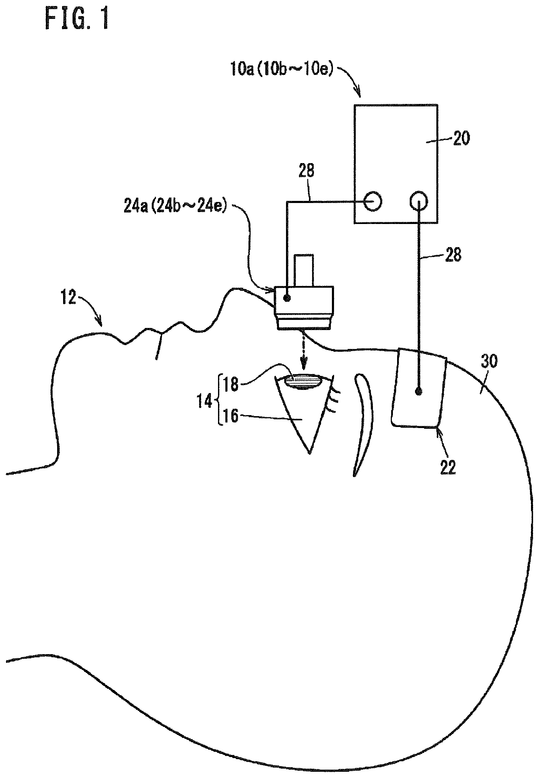

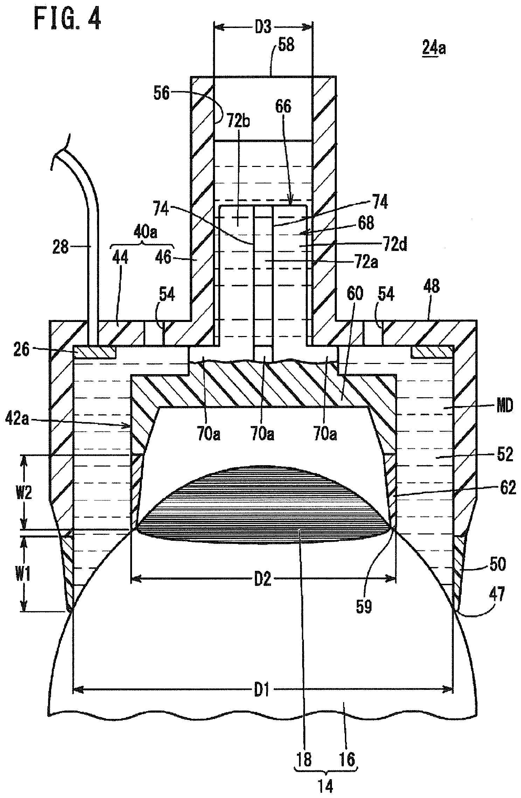

Fig. 1 is a schematic configuration diagram of a transocular iontophoresis device 10a according to a first embodiment. The transocular iontophoresis device 10a is provided for administering medicine MD (see Fig. 4) to an eyeball 14 of a patient 12 using iontophoresis. Note that the eyeball 14 includes a sclera 16 and a cornea 18.

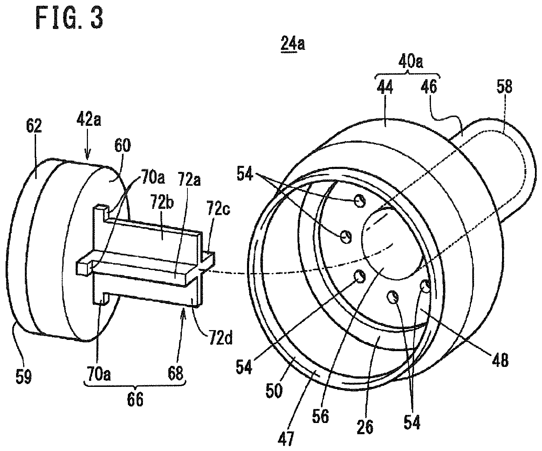

The transocular iontophoresis device 10a includes a power supply controller 20, a passive electrode 22 as a first electrode, and a medicine administration tool 24a. The power supply controller 20 is electrically connected to the passive electrode 22 and an active electrode 26 (particularly, see Fig. 3) as a second electrode provided inside the medicine administration tool 24a via a control line 28. The active electrode 26 is in contact with the medicine MD supplied into the medicine administration tool 24a and is electrically connected to the medicine MD.

The passive electrode 22 is attached to an appropriate site on the head and neck of the patient 12, for example, a forehead 30. On the other hand, the medicine administration tool 24a is disposed on the eyeball 14. In this state, a current flows from the active electrode 26 to the passive electrode 22 or in an opposite direction using the medicine MD supplied into the medicine administration tool 24a and the human body of the patient 12 as conductors. In other words, the passive electrode 22 and the active electrode 26 constitute an electric circuit through the medicine MD and the human body of the patient 12.

As the power supply controller 20, one having a function of controlling a magnitude of the current or voltage flowing through the above-described electric circuit, the polarity of the active electrode 26 and the passive electrode 22, and the power supply form such as a direct current or a pulse current is suitably selected. In particular, a device having a function of energizing a depolarization current in a reverse direction for eliminating a polarized state of a biological tissue with which the medicine administration tool 24a is in contact or the electrodes 22 and 26 is preferably selected. Specifically, the device preferably has a function capable of changing the polarity of power applied to the active electrode 26 and the passive electrode 22. Note that the power supply controller 20 may supply a direct current or may supply an alternating current.

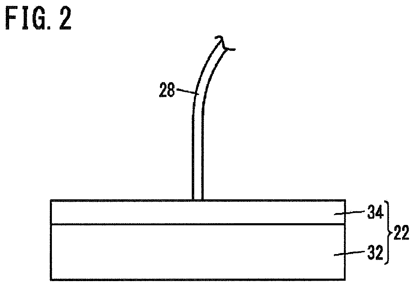

Fig. 2 is an enlarged front view of the passive electrode 22. The passive electrode 22 includes a conductive material layer 32 that is a portion in contact with the skin of the patient 12, and an electrode portion 34 provided on an upper surface (side away from the skin) of the conductive material layer 32. The conductive material layer 32 is made of a material in which an electrolyte such as an inorganic salt is permeated into a water absorbent sheet such as porous polyurethane, a material in which a gel-like material containing an electrolyte such as an inorganic salt is prepared in a sheet shape, or the like. The gel-like material may be a material having adhesive force to the skin. Suitable examples of such an adhesive gel-like material include acrylate polymers.

The electrode portion 34 is formed in a flat plate shape and is provided so as to cover the entire upper surface of the conductive material layer 32. As a constituent material of the electrode portion 34, silver, silver/silver chloride, lead, zinc, tin, titanium, platinum, gold, stainless steel, carbon, or the like, is used. The electrode portion 34 is electrically connected to the power supply controller 20 via the control line 28.

The passive electrode 22 configured as described above is preferably covered with an insulating material except for a skin contact surface of the conductive material layer 32. In addition, the passive electrode 22 may include only the electrode portion 34. In this case, it is preferable to apply an electrolyte solution in advance when the passive electrode 22 (electrode portion 34) is brought into contact with or attached to the skin.

Next, a specific configuration of the medicine administration tool 24a will be described. Figs. 3 and 4 are a schematic exploded perspective view and a schematic longitudinal cross-sectional view of the medicine administration tool 24a, respectively. The medicine administration tool 24a includes an outer cylinder 40a as a tubular member and an inner cylinder 42a as an inner member. Suitable materials of the outer cylinder 40a and the inner cylinder 42a include a hard material such as an acrylic resin. Further, thicknesses (diameter difference between the outer diameter and the inner diameter) of the outer cylinder 40a and the inner cylinder 42a are preferably within a range of 0.5 to 2.0 mm.

The outer cylinder 40a has a first tubular portion 44 and a second tubular portion 46 each having a cylindrical shape. In the first tubular portion 44, a first lower end 47 facing the eyeball 14 is formed as an open end, and a first ceiling wall 48 (upper end wall) is formed at an upper end. The second tubular portion 46 set to have a smaller diameter than the first tubular portion 44 protrudes from the first ceiling wall 48 so as to extend in a direction away from the eyeball 14, that is, upward. Hollows of the first tubular portion 44 and the second tubular portion 46 communicate with each other.

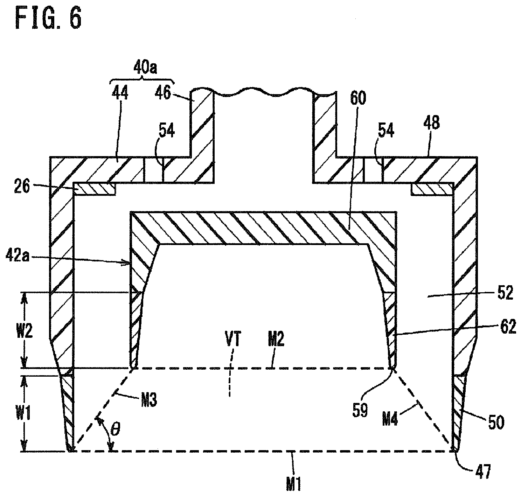

An annular first protective material 50 having an annular shape is joined to a lower edge of the first lower end 47 of the outer cylinder 40a facing the eyeball 14. The first protective material 50 is made of an elastic material, and preferable specific examples thereof include a silicone-based resin and a urethane-based resin. As illustrated in Fig. 6, a lower end edge portion (tip on the eyepiece side) of the first protective material 50 is preferably chamfered so as to be an R surface. In addition, a length W1 of the first protective material 50 in a direction (hereinafter, also referred to as a "height direction" for convenience) parallel to an extending direction of the second tubular portion 46 is preferably within a range of 0.5 to 3 mm.

A medicine accommodating chamber 52 in which the medicine MD is accommodated is formed between an inner peripheral wall of the first tubular portion 44 and an outer peripheral wall of the inner cylinder 42a. The medicine MD is accommodated in the medicine accommodating chamber 52, and the medicine MD is supplied to the sclera 16 in particular of the eyeball 14. The medicine accommodating chamber 52 will be described later.

The active electrode 26 is positioned and fixed on an inner surface of the first ceiling wall 48 of the first tubular portion 44 such that an outer peripheral edge thereof abuts on the inner peripheral wall of the first tubular portion 44. The active electrode 26 has an annular shape, and thus, the active electrode 26 does not block communication between the hollows of the first tubular portion 44 and the second tubular portion 46. In addition, the first tubular portion 44 is provided with a terminal (not illustrated), and the active electrode 26 is electrically connected to the power supply controller 20 via the terminal and the control line 28.

The active electrode 26 is made of, for example, silver, silver/silver chloride, aluminum, zinc, tin, titanium, platinum, gold, stainless steel, carbon, a composite material thereof, or the like, similarly to the passive electrode 22. In particular, titanium, platinum, or gold is suitable because it is chemically stable.

In order to further improve chemical stability of the active electrode 26, the active electrode 26 may be coated with a film. As a material of the coating film, a conductive material is selected which is not likely to elute ions when energization is performed between the passive electrode 22 and the active electrode 26, and the active electrode 26 and the medicine MD are in electrical contact with each other. Suitable specific examples include platinum, gold, thiophene polymers, acetylene polymers, aniline polymers, and pyrrole polymers, but are not particularly limited thereto.

During administration of the medicine MD by iontophoresis, gas may be generated in the active electrode 26. In order to quickly discharge this gas into the atmosphere, a plurality of exhaust holes 54 is formed to penetrate the first ceiling wall 48. The plurality of exhaust holes 54 surrounds the second tubular portion 46 and is positioned so as to be separated from each other at substantially equal intervals (see Fig. 3).

The hollow of the second tubular portion 46 serves as a medicine passage 56 as an upper flow passage. The medicine passage 56 communicates with the medicine accommodating chamber 52. An upper end opening of the second tubular portion 46 serves as a medicine supply port 58 into which the medicine MD is injected (supplied). It is a matter of course that the medicine supply port 58 communicates with the medicine accommodating chamber 52 via the medicine passage 56.

The inner cylinder 42a has a peripheral wall portion in which a second lower end 59 facing the eyeball 14 is formed as an open end, and is a cylindrical body in which an upper end is a closed end by a second ceiling wall 60 (ceiling wall). An annular second protective material 62 having an annular shape is joined to a lower edge of the second lower end 59 of the inner cylinder 42a facing the sclera 16 at a peripheral edge of the cornea 18. Similarly to the first protective material 50, the second protective material 62 is also made of an elastic material, preferably a silicone-based resin, a urethane-based resin, or the like. Further, a lower end edge portion (tip on the eyepiece side) of the second protective material 62 is also preferably an R surface. Further, a height-direction dimension W2 of the second protective material 62 is preferably within a range of 0.5 to 1.5 mm.

If a corneal diameter is CD, an inner diameter D1 of the first tubular portion 44 is preferably within a range where a relationship of 1.4 × CD ≤ D1 ≤ 1.7 × CD is satisfied. On the other hand, an outer diameter D2 of the inner cylinder 42a is preferably within a range where a relationship of CD < D2 ≤ 1.2 × CD is satisfied. Further, each of a dimension in the height direction from a distal end of a lower end portion of the second protective material 62 to an upper end surface of an outer surface of the second ceiling wall 60 of the inner cylinder 42a and a dimension in the height direction from a distal end of a lower end portion of the first protective material 50 to an upper end surface of an outer surface of the first ceiling wall 48 of the outer cylinder 40a may be, for example, 3 mm or more and 4 mm or more.

A space between the second ceiling wall 60 of the inner cylinder 42a and the first ceiling wall 48 of the first tubular portion 44 and a space between the outer peripheral wall of the inner cylinder 42a and the inner peripheral wall of the first tubular portion 44 constitute the medicine accommodating chamber 52 that accommodates the medicine MD. Thus, the active electrode 26 is disposed in the medicine accommodating chamber 52. In addition, the active electrode 26 preferably has an outer diameter substantially equal to D1 and an inner diameter substantially equal to D2. This is because, in this case, the active electrode 26 sufficiently contacts the medicine MD flowing through the medicine accommodating chamber 52.

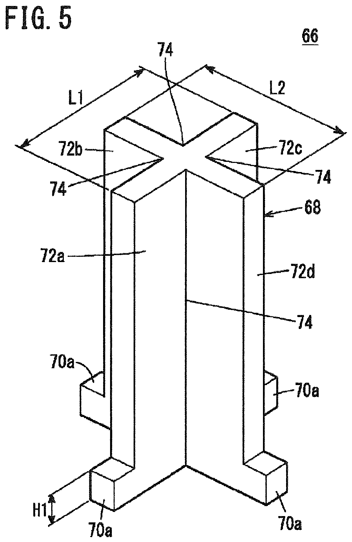

In the first embodiment, a bush 66 as a support member is positioned and fixed to the second ceiling wall 60 of the inner cylinder 42a. As illustrated in Figs. 3 and 5, the bush 66 includes a cross-shaped portion 68 and a stopper portion 70a. The cross-shaped portion 68 has a shape in which one end portions of four columnar bodies radially extending from the center of the inner cylinder 42a are connected to each other. Thus, the cross-shaped portion 68 has four sides of a first side portion 72a, a second side portion 72b, a third side portion 72c, and a fourth side portion 72d. A groove 74 is formed between adjacent side portions, that is, on an outer periphery of the cross-shaped portion 68.

The cross-shaped portion 68 is inserted into the hollow (medicine passage 56) of the second tubular portion 46. Here, an extending length of the cross-shaped portion 68 is set to be smaller than an extending length of the second tubular portion 46. Thus, the entire cross-shaped portion 68 is accommodated in the second tubular portion 46. Note that the medicine MD supplied from the medicine supply port 58 can pass through the medicine passage 56 by flowing through the groove 74. In other words, the groove 74 allows the medicine MD to pass therethrough.

As illustrated in Fig. 5, a length L1 from an outer edge of the first side portion 72a of the cross-shaped portion 68 to an outer edge of the third side portion 72c facing the first side portion 72a and a length L2 from an outer edge of the second side portion 72b to an outer edge of the fourth side portion 72d facing the second side portion 72b are set to be slightly smaller than an inner diameter D3 of the second tubular portion 46 (see Fig. 4). Thus, the cross-shaped portion 68 is not restrained from the inner peripheral wall of the second tubular portion 46. Thus, the cross-shaped portion 68 can move inside the second tubular portion 46 while being guided by the second tubular portion 46. Accordingly, the inner cylinder 42a moves along the extending direction of the second tubular portion 46, in other words, the height direction. Note that L1 and L2 are equivalent.

On the other hand, the stopper portion 70a protrudes from the end portions of the first side portion 72a to the fourth side portion 72d facing the inner cylinder 42a. A protruding direction of the stopper portion 70a is the same as a protruding direction of the first side portion 72a to the fourth side portion 72d. Each of the four stopper portions 70a is part of the bush 66 continuous so as to form a step portion with respect to the first side portion 72a to the fourth side portion 72d and radially extends.

As illustrated in Fig. 3, the stopper portion 70a is engaged between the second ceiling wall 60 of the inner cylinder 42a and the first ceiling wall 48 of the first tubular portion 44 and prevents further movement of the inner cylinder 42a toward the second tubular portion 46. If a dimension H1 in the height direction of the stopper portion 70a is excessively small, a cross-sectional area between the second ceiling wall 60 and the first ceiling wall 48 becomes small in a side view of the medicine administration tool 24a, and passing speed of the medicine MD becomes low. In order to avoid this, H1 is preferably 1 mm or more.

Here, as illustrated in Fig. 6, in a side view of the medicine administration tool 24a, a first virtual line M1 and a second virtual line M2 are drawn along an opening diameter (opening width) of the first protective material 50 and an opening width (opening diameter) of the second protective material 62, respectively. A length of each of the first virtual line M1 and the second virtual line M2 is substantially the same as the outer diameter of the first tubular portion 44 and the inner diameter of the inner cylinder 42a. Further, a third virtual line M3 and a fourth virtual line M4 are respectively drawn at end portions of the first virtual line M1 and the second virtual line M2 close to each other. As described above, a virtual trapezoid VT having the first virtual line M1 as a lower side, the second virtual line M2 as an upper side, and the third virtual line M3 and the fourth virtual line M4 as oblique sides is formed.

In the virtual trapezoid VT, an intersection angle θ between the first virtual line M1 and the third virtual line M3 changes as the inner cylinder 42a moves. The dimensions of the cross-shaped portion 68 and the stopper portion 70a in the height direction are preferably set such that the intersection angle θ before movement of the inner cylinder 42a exceeds 0°, and the intersection angle θ when movement of the inner cylinder 42a is stopped by the stopper portion 70a becomes less than 80°. In other words, the inner cylinder 42a is preferably movable with respect to the outer cylinder 40a within a range where the intersection angle θ is more than 0° and less than 80°. This is because even when the degree of curvature of the eyeball 14 differs on the basis of individual differences, it is possible to cover the cornea 18 with the second protective material 62 and cover the sclera 16 with the first protective material 50 at the same time.

Here, the medicine MD is a solution in which medicine is dissolved in a solvent. In the solvent, an ionic substance may be further dissolved in order to enhance conductivity. Suitable examples of the ionic substance include citric acid or a salt thereof, phosphoric acid or a salt thereof, and boric acid or a salt thereof. On the other hand, suitable examples of the medicine include an antibody and a nucleic acid pharmaceutical product as described on pages 88 to 102 of Bulletin No. 23 of Suzuki Medical Science University, 2016. Among them, an anti-VEGF antibody such as LUCENTIS (ranibizumab), a VEGF inhibitor such as EYLEA (aflibercept) or MACGEN (pegabutanib sodium) which is siRNA of a nucleic acid medicine, and the like, are ionic medicine and can be used as the medicine MD without separately adding an ionic substance as described above to a solvent.

The transocular iontophoresis device 10a according to the first embodiment is basically configured as described above, and operation and effect thereof will be described below.

In order to administer the medicine MD to the eyeball 14 of the patient 12 using the transocular iontophoresis device 10a, after the patient 12 is placed on his/her back, as illustrated in Fig. 1, the passive electrode 22 is attached to the skin such as the forehead 30, and the medicine administration tool 24a is placed on the eyeball 14. In this event, first, the inner cylinder 42a is disposed on the cornea 18. As a result, the second protective material 62 comes into contact with the cornea 18 so as to surround (cover) the peripheral edge portion of the cornea 18. The second protective material 62 is made of a soft elastic material and the lower end edge portion thereof is the R surface, so that it is avoided that the patient 12 feels a tender feeling as the second protective material 62 comes into contact with the cornea 18. In the middle of lowering the inner cylinder 42a, the outer cylinder 40a may be held at a predetermined position separated from the eyeball 14 and at which the bush 66 is not separated from the second tubular portion 46, for example, by being held by a user with a finger.

Next, the outer cylinder 40a is lowered while the bush 66 guides the second tubular portion 46, and the eyeball 14 is covered with the second tubular portion 46. In this event, the first protective material 50 is in contact with the sclera 16. Here, the first protective material 50 is made of a soft elastic material similarly to the second protective material 62, and the lower end edge portion thereof is the R surface. It is therefore avoided that the patient 12 feels a tender feeling as the first protective material 50 comes into contact with the sclera 16.

As described above, according to the first embodiment, when the medicine administration tool 24a is placed on the eyeball 14 and the first protective material 50 to the second protective material 62 are brought into contact with the eyeball 14, the tender feeling of the patient 12 is alleviated. This reduces psychological burden on the patient 12.

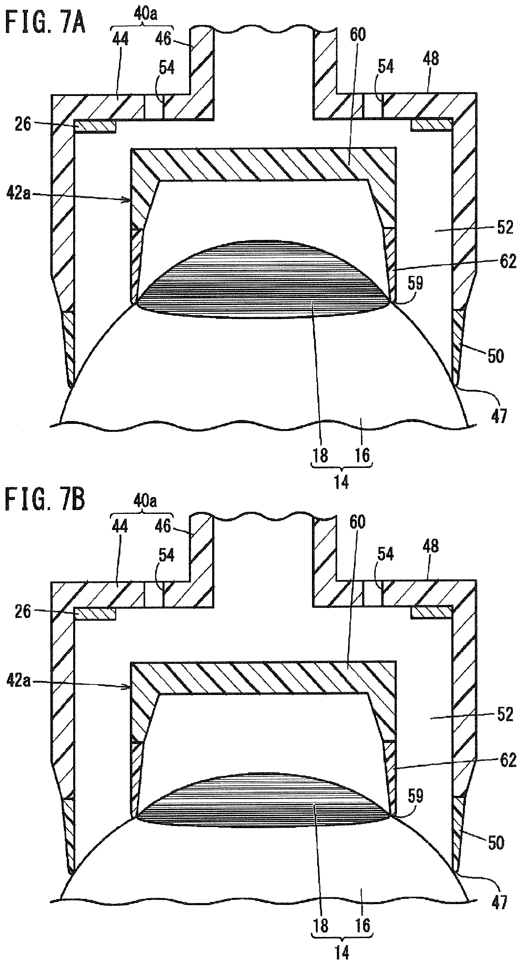

As illustrated in Figs. 7A and 7B, the degree of curvature of the eyeball 14 differs depending on the patient 12. In such a case, a lowering distance of the outer cylinder 40a is different. Specifically, in a case where the degree of curvature of the eyeball 14 is large, the lowering distance of the outer cylinder 40a increases, and as a result, as illustrated in Fig. 7A, a distance between the first lower end 47 of the first tubular portion 44 and the second lower end 59 of the inner cylinder 42a increases. On the other hand, in a case where the degree of curvature of the eyeball 14 is small, the lowering distance of the outer cylinder 40a decreases, and as illustrated in Fig. 7B, the distance between the first lower end 47 of the first tubular portion 44 and the second lower end 59 of the inner cylinder 42a decreases.

As described above, in the present embodiment, the outer cylinder 40a can move relative to the inner cylinder 42a. Thus, after the second protective material 62 of the inner cylinder 42a comes into contact with the periphery of the cornea 18, a moving distance until the first protective material 50 of the outer cylinder 40a comes into contact with the sclera 16 can be made different. In addition, the lower ends of the first protective material 50 and the second protective material 62 are bent according to the degree of curvature of the eyeball 14 so as to spread outward in a diametrical direction by the weight of the outer cylinder 40a or the inner cylinder 42a. Thus, regardless of the degree of curvature of the eyeball 14, the first protective material 50 can be brought into contact with the sclera 16 after the second protective material 62 comes into contact with the cornea 18.

Moreover, the second lower end 59 of the inner cylinder 42a and the first lower end 47 of the first tubular portion 44 approach each other or are separated from each other such that the intersection angle θ illustrated in Fig. 6 falls within a range of more than 0° to less than 80°. In other words, a movable distance of the outer cylinder 40a relative to the inner cylinder 42a is large. It is therefore possible to support eyeballs 14 with various degrees of curvature. In other words, the medicine administration tool 24a has high versatility. As described above, by making the outer cylinder 40a and the inner cylinder 42a relatively movable, the first protective material 50 and the second protective material 62 constituting the medicine administration tool 24a can be brought into close contact with the eyeball 14 without being affected by the degree of curvature (individual difference in shape) of the eyeball 14.

In a case where the outer cylinder 40a greatly descends with respect to the inner cylinder 42a, the stopper portion 70a provided in the bush 66 abuts on the first ceiling wall 48 of the first tubular portion 44. Due to this abutment, the outer cylinder 40a is blocked, which prevents further relative rising of the inner cylinder 42a. In other words, movement of the inner cylinder 42a toward the second tubular portion 46 is stopped.

Next, while the power supply controller 20 is activated, the liquid medicine MD is supplied from the medicine supply port 58 of the second tubular portion 46. The power supply controller 20 performs iontophoresis by controlling current density to be less than 10 mA/cm², for example.

The medicine MD flows through the medicine passage 56 formed in the second tubular portion 46 and enters the hollow of the first tubular portion 44. In this event, there is a clearance between adjacent sides of the cross-shaped portion 68 and between the outer edge of the cross-shaped portion 68 and the inner peripheral wall of the second tubular portion 46. Thus, the medicine passage 56 is not blocked by the bush 66, and circulation of the medicine MD is not hindered.

Note that the upper end of the inner cylinder 42a is a closed end that is closed by the second ceiling wall 60 as described above. Thus, the medicine MD flows into the medicine accommodating chamber 52 formed between the outer peripheral wall of the inner cylinder 42a and the inner peripheral wall of the first tubular portion 44 without entering the hollow of the inner cylinder 42a. The medicine MD further reaches the sclera 16. In this event, the first protective material 50 is in contact with the sclera 16, and thus, the medicine MD is prevented from leaking from the clearance between the first protective material 50 and the sclera 16 to outside of the medicine administration tool 24a. In addition, the second protective material 62 is in contact with the peripheral edge of the cornea 18, and thus, the medicine MD is also prevented from passing through the clearance between the second protective material 62 and the cornea 18 and reaching the hollow of the inner cylinder 42a, that is, the cornea 18.

The medicine MD has conductivity by, for example, adding an ionic substance such as citric acid or a salt thereof, phosphoric acid or a salt thereof, or boric acid or a salt thereof, or dissolving ionic medicine such as an anti-VEGF antibody such as Lucentis (ranibizumab), or a VEGF inhibitor such as Eylea (aflibercept) or Macgen (pegabutanib sodium) which is siRNA of nucleic acid medicine. Further, as described above, the passive electrode 22 and the active electrode 26 constitute an electric circuit via the human body of the patient 12. The power supply controller 20 switches the polarity of the active electrode 26 to positive when a charge of the medicine MD is positive and to negative when a charge of the medicine MD is negative and applies a current to the medicine MD. The resulting electrical repulsion or electroosmotic flow causes the medicine MD to penetrate from the sclera 16 into inside of the eyeball 14. The medicine MD stays in the medicine accommodating chamber 52 as described above, and thus, an active ingredient of the medicine MD enters the eyeball 14 in a sufficient amount.

As described above, according to the transocular iontophoresis device 10a of the first embodiment, a sufficient amount of the medicine MD can be administered to the eyeball 14 which is the affected part. Moreover, psychological burden on the patient 12 can be reduced. Furthermore, the cornea 18 and the sclera 16 are partitioned by the inner cylinder 42a and the second protective material 62, so that the medicine MD is prevented from reaching the cornea 18 and the current is prevented from flowing to the cornea 18 through the medicine MD. Thus, in particular, the cornea 18 is protected.

In a case where gas is generated in the active electrode 26, the gas is discharged to the atmosphere through the exhaust hole 54 formed in the first ceiling wall 48 of the first tubular portion 44. Thus, the gas is avoided from staying in the medicine MD.

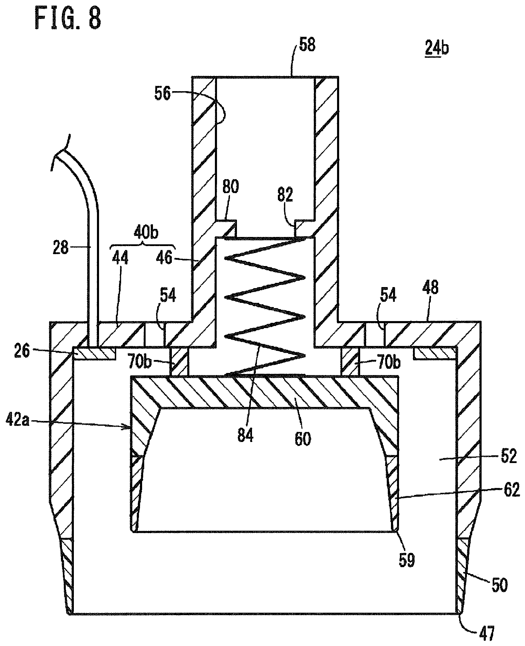

Next, a medicine administration tool 24b constituting a transocular iontophoresis device 10b according to a second embodiment will be described with reference to Fig. 8. Note that the same components as those illustrated in Figs. 1 to 7 are denoted by the same reference numerals, and detailed description thereof will be omitted.

In this case, four stopper portions 70b made of a quadrangular prism-shaped block body are radially positioned and fixed to the outer surface of the second ceiling wall 60 of the inner cylinder 42a. A dimension of the stopper portion 70b in the height direction is preferably 1 mm or more for the same reason as in the first embodiment. Examples of a suitable material of the stopper portion 70b can include a hard material such as an acrylic resin similarly to the outer cylinder 40b and the inner cylinder 42a.

An annular seating portion 80 is provided in the second tubular portion 46 of the outer cylinder 40b. In other words, a through hole 82 is formed at the center of the seating portion 80. The medicine MD supplied from the medicine supply port 58 into the second tubular portion 46 passes through the through hole 82 and flows into the medicine accommodating chamber 52. In other words, circulation of the medicine MD is not hindered by formation of the through hole 82.

One end of a coil spring 84 that is an elastic body is seated on an outer surface of the second ceiling wall 60 of the inner cylinder 42a. Preferably, one end of the coil spring 84 is connected to the outer surface of the second ceiling wall 60 of the inner cylinder 42a. On the other hand, another end of the coil spring 84 is seated on the seating portion 80. Thus, the inner cylinder 42a is supported in a floating manner with respect to the outer cylinder 40b via the coil spring 84. Here, examples of a suitable material of the coil spring 84 include a metal such as stainless steel and a resin (plastic). In a case of a resin, polyethylene (PE), polypropylene (PP), or polycarbonate (PC) may be used, but polyoxymethylene (POM) or polyetheretherketone (PEEK) is still more preferable.

In the transocular iontophoresis device 10b according to the second embodiment, as illustrated in Fig. 1, the passive electrode 22 is attached to the skin such as the forehead 30 of the patient 12, and the medicine administration tool 24b is placed on the eyeball 14. In this event, substantially at the same time as the first protective material 50 comes into contact with the sclera 16, the second protective material 62 comes into contact with the cornea 18. Here, in a case where the degree of curvature of the eyeball 14 is large, the inner cylinder 42a rises relative to the outer cylinder 40b by the coil spring 84 being compressed. Thus, the inner cylinder 42a and the outer cylinder 40b are relatively movable by supporting the inner cylinder 42a in a floating manner with respect to the outer cylinder 40b.

Thereafter, similarly to the first embodiment, the medicine MD is administered via the eyeball 14 using iontophoresis. In other words, in the second embodiment, the same effects as those of the first embodiment can be obtained. In addition, according to the second embodiment, it is not necessary to individually place the outer cylinder 40b and the inner cylinder 42a on the eyeball 14, so that there is an advantage that the operation is simplified accordingly.

Similarly to the first embodiment, the inner cylinder 42a and the outer cylinder 40b may be individually placed on the eyeball 14. In this case, the second protective material 62 of the inner cylinder 42a is first brought into contact with the cornea 18, and then the outer cylinder 40b may be put on the inner cylinder 42a such that the coil spring 84 enters the hollow (medicine passage 56) of the second tubular portion 46.

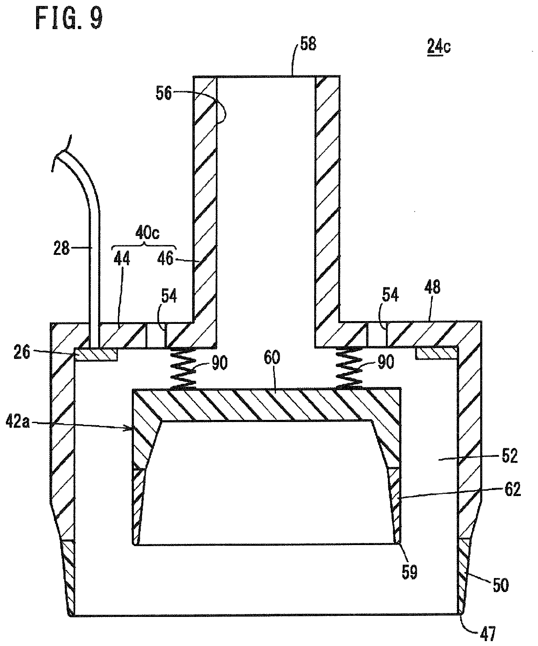

Next, a medicine administration tool 24c constituting a transocular iontophoresis device 10c according to a third embodiment will be described with reference to Fig. 9. Note that the same components as those illustrated in Figs. 1 to 7 are denoted by the same reference numerals, and detailed description thereof will be omitted.

In this case, a plurality of small coil springs 90 is disposed as an elastic body between the second ceiling wall 60 of the inner cylinder 42a and the first ceiling wall 48 of the outer cylinder 40c. In other words, one end of the small coil spring 90 is seated so as to be attached to the second ceiling wall 60 of the inner cylinder 42a, and the other end is seated on the first ceiling wall 48 of the outer cylinder 40c. As a result, the inner cylinder 42a is supported in a floating manner with respect to the outer cylinder 40c. The plurality of small coil springs 90 is located on an inner peripheral side of a virtual circle connecting the plurality of exhaust holes 54 and is arranged on another virtual circle concentric with the virtual circle.

As the small coil spring 90, a small coil spring having a height direction dimension of 1 mm or more when maximally contracted is suitably selected. Thus, a clearance of 1 mm or more is formed between the second ceiling wall 60 of the inner cylinder 42a and the first ceiling wall 48 of the outer cylinder 40c. Thus, the medicine accommodating chamber 52 is not blocked when the small coil spring 90 contracts. In addition, examples of a suitable material of the small coil spring 90 include a metal such as stainless steel and a resin (plastic) such as PE, 22, PC, POM, or PEEK as with the coil spring 84.

In a case of using the transocular iontophoresis device 10c according to the third embodiment, as illustrated in Fig. 1, the passive electrode 22 is attached to the skin such as the forehead 30 of the patient 12, and the medicine administration tool 24c is placed on the eyeball 14. In this event, substantially at the same time as the first protective material 50 comes into contact with the sclera 16, the second protective material 62 comes into contact with the cornea 18. Here, in a case where the degree of curvature of the eyeball 14 is large, the inner cylinder 42a rises relative to the outer cylinder 40c by the small coil spring 90 being compressed. In other words, also in the third embodiment, the inner cylinder 42a and the outer cylinder 40c are relatively movable by supporting the inner cylinder 42a in a floating manner with respect to the outer cylinder 40c.

In the third embodiment, the small coil spring 90 also functions as a stopper portion. In other words, when the small coil spring 90 contracts the most, further relative movement of the inner cylinder 42a with respect to the outer cylinder 40c is stopped.

After the small coil spring 90 contracts according to the degree of curvature of the eyeball 14, the medicine MD is administered via the eyeball 14 using iontophoresis as in the first embodiment and the second embodiment. In other words, in the third embodiment, the same effects as those of the first embodiment and the second embodiment can be obtained. Also in the third embodiment, it is not necessary to place the outer cylinder 40c and the inner cylinder 42a individually on the eyeball 14, so that operation is simplified accordingly. However, as in the first embodiment, the inner cylinder 42a and the outer cylinder 40c may be individually placed on the eyeball 14.

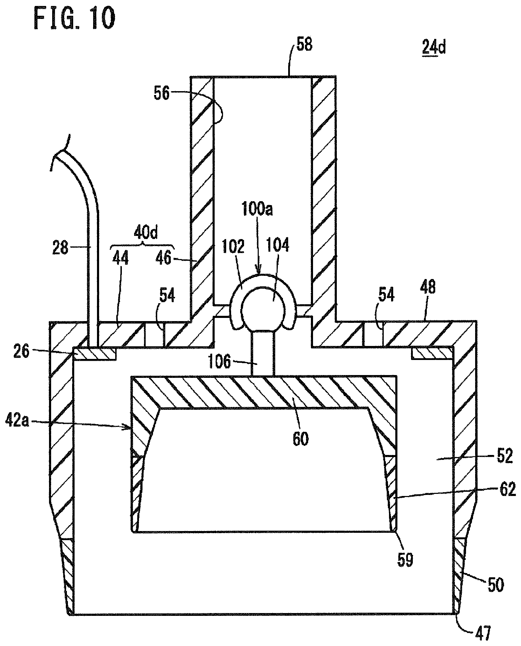

Next, a medicine administration tool 24d constituting a transocular iontophoresis device 10d according to a fourth embodiment will be described with reference to Fig. 10. In the medicine administration tool 24d, the inner cylinder 42a is supported by an outer cylinder 40d via a ball joint 100a.

The ball joint 100a includes a ball receiving portion 102 and a swing shaft 106 provided with a rolling ball portion 104 at the tip. The ball receiving portion 102 is supported in the hollow (medicine passage 56) of the second tubular portion 46, and the rolling ball portion 104 is rollably included in the ball receiving portion 102. On the other hand, the tip of the swing shaft 106 is connected to the second ceiling wall 60 of the inner cylinder 42a. Thus, as the rolling ball portion 104 rolls in the ball receiving portion 102, the swing shaft 106 and the inner cylinder 42a integrally swing (move) in the hollow of the outer cylinder 40d.

More specifically, the swing shaft 106 and the inner cylinder 42a swing with the rolling ball portion 104 as a swing center. In other words, the inner cylinder 42a and the second protective material 62 can be inclined with respect to the outer cylinder 40d. Thus, for example, even in a case where there is an individual difference in the position of the cornea 18, the positions of the inner cylinder 42a and the second protective material 62 can be appropriately adjusted according to a position of the cornea 18 of the patient 12.

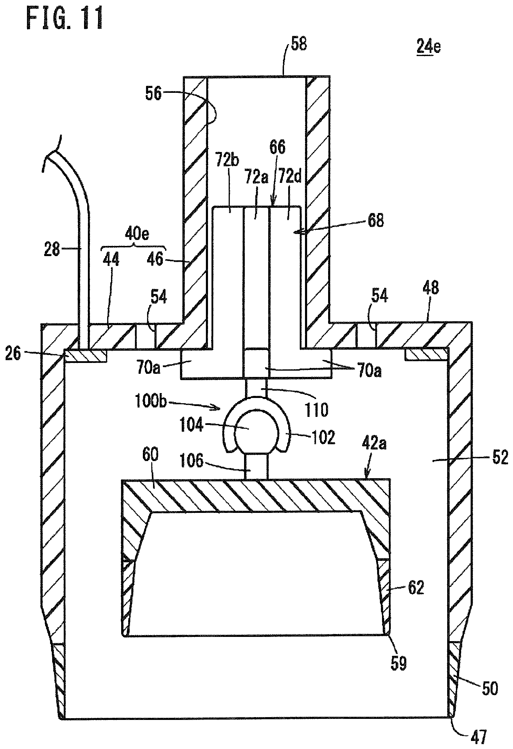

For example, as illustrated in Fig. 11, the bush 66 and a ball joint 100b may be combined. This will be described as a medicine administration tool 24e constituting a transocular iontophoresis device 10e according to a fifth embodiment.

In this case, a bottomed screw hole is provided on a lower end surface of the bush 66 constituting the medicine administration tool 24e. On the other hand, the ball joint 100b includes a screw shaft 110, the ball receiving portion 102 provided on the screw shaft 110, and the swing shaft 106 provided with a rolling ball portion 104. The screw shaft 110 among these is screwed into the screw hole, and the lower end of the swing shaft 106 is attached to the second ceiling wall 60 of the inner cylinder 42a. As a result, the bush 66 is provided in the inner cylinder 42a via the ball joint 100b.

As in the first embodiment, the bush 66 can easily move (ascend and descend) in the hollow of the second tubular portion 46. Further, the inner cylinder 42a swings with the rolling ball portion 104 as a swing center under action of the ball joint 100b. In other words, according to the fifth embodiment, inclination of the inner cylinder 42a can be adjusted according to individual differences in the position of the cornea 18, and a relative height position of the inner cylinder 42a with respect to the outer cylinder 40e can be adjusted according to individual differences in the curvature of the eyeball 14.

The present invention is not particularly limited to the first to fifth embodiments described above, and various modifications can be made without departing from the gist of the present invention.

For example, the first protective material 50 and the second protective material 62 do not particularly need to have an annular shape and may be formed in an annular shape in accordance with the shapes of the outer cylinders 40a to 40e and the inner cylinder 42a.

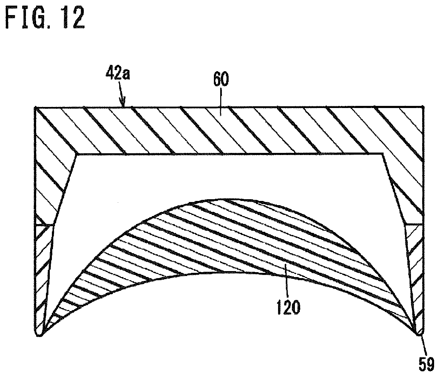

As the second protective material, a film-shaped body 120 illustrated in Fig. 12 may be adopted instead of the annular shape body illustrated in Figs. 3 to 11. In this case, the film-shaped body 120 covers the entire cornea 18 while being elastically deformed along the shape of the cornea 18. In other words, the cornea 18 is protected by the film-shaped body 120 elastically deformed according to the shape of the cornea 18.

Note that from the viewpoint of protecting the cornea 18 from electricity, the film-shaped body 120 is preferably an insulator. In other words, a material of the film-shaped body 120 is preferably one that can protect the cornea 18 and exhibits insulating properties. Typical examples of this type of material include a methacrylate-based resin, a silicone-based resin, and an acryl-based resin, which are materials for contact lenses.

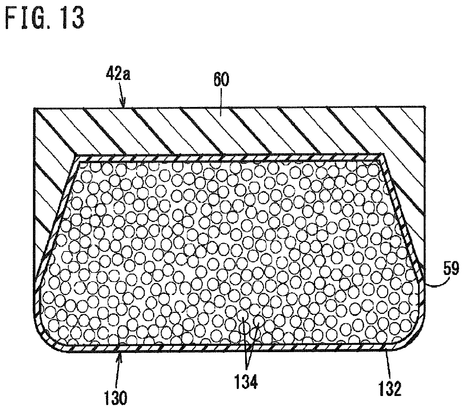

A bead cushion 130 illustrated in Fig. 13 may be used as the second protective material. In this configuration, the bead cushion 130 is a buffer body containing a large amount of soft beads 134 in an insulating film 132. When the bead cushion 130 comes into contact with the cornea 18, the insulating film 132 is flexibly deformed as the soft beads 134 move according to the shape of the cornea 18. In other words, the cornea 18 is protected by the bead cushion 130 deformed according to reaction force from the cornea 18.

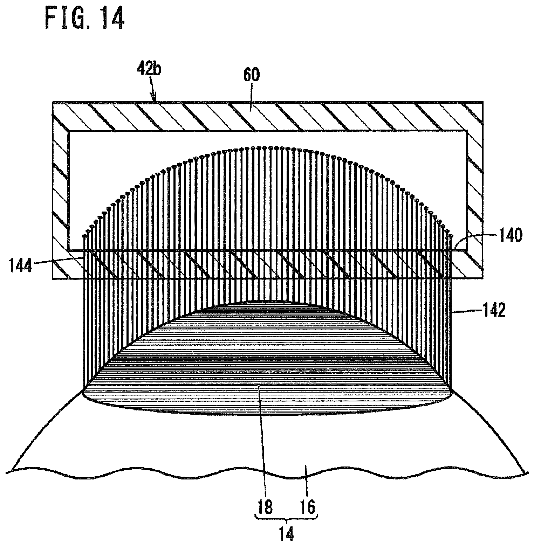

As illustrated in Fig. 14, a second lower end of the inner cylinder 42b may be a bottom wall 140 having a screen mesh shape, and part of a soft polymer 142 (for example, polymer gel) accommodated in the hollow may be the second protective material. In this case, the lower end of the soft polymer 142 passes through a net 144 formed on the bottom wall 140 and is exposed from the inner cylinder 42b. On the other hand, an upper end is set to be wider than the net 144, thereby preventing the soft polymer 142 from coming off. Alternatively, the upper ends may be integrated with each other.

In this case, if the lower end of the soft polymer 142 exposed from the net 144 comes into contact with the cornea 18, the soft polymer 142 is pressed toward the inner cylinder 42b. As a result, the upper end side of the soft polymer 142 enters the hollow of the inner cylinder 42b. Here, a degree of entry into the hollow on the upper end side varies depending on the degree of curvature of the cornea 18. In other words, as the degree of curvature of the cornea 18 is larger, the degree of entry of the soft polymer 142 into the hollow on the upper end side becomes larger. Of course, this is the opposite in a case where the degree of curvature of the cornea 18 is small.

As described above, the soft polymer 142 is deformed according to the degree of curvature of the cornea 18. Thus, the cornea 18 is covered with the soft polymer 142 and thus protected.

Note that in a case where the first protective material 50 and the second protective material 62 are not provided, the first virtual line M1 may be drawn along an opening width of the first tubular portion 44, and the second virtual line M2 may be drawn along an opening width of the inner cylinder 42a located in the first tubular portion 44. When the virtual trapezoid VT is obtained by drawing the third virtual line M3 and the fourth virtual line M4 in a similar manner to in Fig. 6, it is preferable that the inner cylinder 42a is movable inside the hollows of the outer cylinders 40a to 40e so that the intersection angle between the first virtual line M1 and the third virtual line M3 falls within a range of more than 0° and less than 80°.

## Claims

1. A medicine administration tool to be used in a transocular iontophoresis device that forms an electric circuit between a first electrode and a second electrode and supplies medicine to an eyeball, the medicine administration tool comprising:
a tubular member having a first lower end facing the eyeball;
an inner member having a second lower end facing the eyeball, the inner member being accommodated in a hollow of the tubular member and forming a medicine accommodating chamber for accommodating the medicine between the inner member and the tubular member; and
a support member movably supporting the inner member with respect to the tubular member.

2. The medicine administration tool according to claim 1,
wherein the support member is configured to be able to move the inner member with respect to the tubular member in an axial direction of the tubular member.

3. The medicine administration tool according to claim 2, wherein the tubular member includes:
a first tubular portion that accommodates the inner member; and
a second tubular portion that is formed to be thinner than the first tubular portion and protrudes upward from an upper end wall of the first tubular portion,
the second tubular portion includes a medicine supply port and a medicine passage that allows communication between the medicine supply port and the medicine accommodating chamber,
the support member includes a bush that is to be inserted into the second tubular portion so as to be movable in the axial direction,
a groove that allows the medicine to pass is formed in an inner peripheral portion of the second tubular portion or an outer peripheral portion of the bush,
the inner member is movable relative to the tubular member along an extending direction of the second tubular portion, and
the medicine administration tool includes a stopper portion that stops movement of the inner member toward the second tubular portion.

4. The medicine administration tool according to claim 3, wherein the stopper portion is part of the bush.

5. The medicine administration tool according to claim 1,
wherein the support member is configured to be able to change a posture of the inner member with respect to the tubular member.

6. The medicine administration tool according to claim 2 or 5,
wherein the support member is an elastic body having one end seated on the inner member and another end seated on the tubular member and supporting the inner member on the tubular member in a floating manner, and the inner member moves so as to approach or separate from an upper end wall of the tubular member as the elastic body expands and contracts.

7. The medicine administration tool according to claim 6,
wherein the inner member or the tubular member is provided with a stopper portion that stops movement of the inner member toward the tubular member.

8. The medicine administration tool according to claim 6,
wherein the elastic body is disposed in the medicine accommodating chamber.

9. The medicine administration tool according to claim 5,
wherein the support member is a ball joint, and the inner member is able to swing with the ball joint as a swing center.

10. The medicine administration tool according to claim 5, wherein the tubular member includes:
a first tubular portion that accommodates the inner member; and
a second tubular portion that is formed to be thinner than the first tubular portion and protrudes upward from an upper end wall of the first tubular portion,
the second tubular portion includes a medicine supply port and a medicine passage that allows communication between the medicine supply port and the medicine accommodating chamber,
the support member includes a bush that is to be inserted into the second tubular portion so as to be movable in the axial direction and a ball joint provided at the bush, and
the inner member is movable relative to the tubular member along an extending direction of the second tubular portion and is able to swing with the ball joint as a swing center.

11. The medicine administration tool according to any one of claims 1 to 10,
wherein a first protective material and a second protective material are respectively provided at the first lower end and the second lower end, and the second protective material is accommodated in the hollow of the tubular member.

12. The medicine administration tool according to claim 11 dependent on claim 3,
wherein the second protective material is annular, and
when a virtual trapezoid is formed by a first virtual line drawn along an opening width of the first protective material, a second virtual line drawn along an opening width of the second protective material located in the first tubular portion, and a third virtual line and a fourth virtual line connecting end portions of the first virtual line and the second virtual line close to each other in a side view of the medicine administration tool, the inner member is movable with respect to the tubular member within a range in which an intersection angle between the first virtual line and the third virtual line or the fourth virtual line is more than 0° and less than 80° .

13. The medicine administration tool according to claim 11,
wherein the second protective material is a film-shaped body covering the entire cornea.

14. The medicine administration tool according to claim 11,
wherein the second protective material is a buffer containing beads.

15. The medicine administration tool according to claim 1,
wherein the inner member includes an annular peripheral wall portion and a ceiling wall that is provided at an upper end of the peripheral wall portion and closes an upper end of the inner member.

16. The medicine administration tool according to claim 15,
wherein the tubular member includes:
a first tubular portion that accommodates the inner member; and
a second tubular portion that is formed to be thinner than the first tubular portion and protrudes upward from an upper end wall of the first tubular portion, and
the second tubular portion includes a medicine supply port and a medicine passage that allows communication between the medicine supply port and the medicine accommodating chamber.

17. The medicine administration tool according to claim 16,
wherein the second tubular portion is located immediately above the inner member.

18. The medicine administration tool according to claim 16 or 17,
wherein the second electrode is provided on an inner surface of the first tubular portion.

19. The medicine administration tool according to claim 1,
wherein when a virtual trapezoid is formed by a first virtual line drawn along an opening width of the tubular member, a second virtual line drawn along an opening width of the inner member located in the tubular member, and a third virtual line and a fourth virtual line connecting end portions of the first virtual line and the second virtual line close to each other in a side view of the medicine administration tool, the inner member is movable with respect to the tubular member within a range in which an intersection angle between the first virtual line and the third virtual line or the fourth virtual line is more than 0° and less than 80°.

20. The medicine administration tool according to any one of claims 1 to 19,
wherein an exhaust hole for discharging a gas in the medicine accommodating chamber to outside of the tubular member is formed in the tubular member.
